(19) **Europäisches Patentamt / European Patent Office / Office européen des brevets**

(11) **EP 2 002 001 B1**

(12) # EUROPEAN PATENT SPECIFICATION

(45) Date of publication and mention
of the grant of the patent:
**13.03.2013 Bulletin 2013/11**

(21) Application number: **06835675.7**

(22) Date of filing: **21.12.2006**

(51) Int Cl.:
*C12N 15/10* (2006.01)     *C12Q 1/68* (2006.01)

(86) International application number:
**PCT/NL2006/000653**

(87) International publication number:
**WO 2007/073170 (28.06.2007 Gazette 2007/26)**

(54) **IMPROVED TARGETED NUCLEOTIDE EXCHANGE WITH LNA MODIFIED OLIGONUCLEOTIDES**

VERBESSERTER GEZIELTER NUKLEOTIDAUSTAUSCH MIT LNA-MODIFIZIERTEN OLIGONUKLEOTIDEN

ECHANGE AMELIORE CIBLE DE NUCLEOTIDES AVEC DES OLIGONUCLEOTIDES MODIFIES PAR LNA

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.12.2005 PCT/NL2005/000884**
**09.05.2006 PCT/NL2006/000244**

(43) Date of publication of application:
**17.12.2008 Bulletin 2008/51**

(73) Proprietor: **KEYGENE N.V.**
**6708 PW Wageningen (NL)**

(72) Inventors:
- **BUNDOCK, Paul**
  **1391 XW Abcoude (NL)**
- **DE BOTH, Michiel, Theodoor, Jan**
  **NL-6708 BG Wageningen (NL)**
- **HOGERS, René, Cornelis, Josephus**
  **NL-6715 GR Ede (NL)**
- **WACHOWSKI, Ludvik, Kevin**
  **NL-6702 BV Wageningen (NL)**

(74) Representative: **Raggers, René John**
**Exter Polak & Charlouis B.V.**
**P.O. Box 3241**
**2280 GE Rijswijk (NL)**

(56) References cited:
**WO-A-01/73002**     **WO-A-01/92512**
**WO-A1-2007/073154**

- **PAREKH-OLMEDO HETAL ET AL: "Targeted nucleotide exchange in Saccharomyces cerevisiae directed by short oligonucleotides containing locked nucleic acids." CHEMISTRY AND BIOLOGY (CAMBRIDGE), vol. 9, no. 10, October 2002 (2002-10), pages 1073-1084, XP002424170 ISSN: 1074-5521**
- **LATORRA D ET AL: "Design considerations and effects of LNA in PCR primers", MOLECULAR AND CELLULAR PROBES, ACADEMIC PRESS, LONDON, GB, vol. 17, no. 5, 1 October 2003 (2003-10-01), pages 253-259, XP004466447, ISSN: 0890-8508, DOI: DOI:10.1016/S0890-8508(03)00062-8**
- **FRIEDEN M ET AL: "Expanding the design horizon of antisense oligonucleotides with alpha-L-LNA", NUCLEIC ACIDS RESEARCH, OXFORD UNIVERSITY PRESS, SURREY, GB, vol. 31, no. 21, 1 November 2003 (2003-11-01), pages 6365-6372, XP002281376, ISSN: 0305-1048, DOI: DOI:10.1093/NAR/GKG820**

**Description**

Field of the invention

The present invention relates to a method for the specific and selective alteration of a nucleotide sequence at a specific site of the DNA in a target cell by the introduction into that cell of an oligonucleotide. The result is the targeted exchange of one or more nucleotides so that the sequence of the target DNA is converted to that of the oligonucleotide where they are different. More in particular, the invention relates to the targeted nucleotide exchange using modified oligonucleotides. The invention also relates to the application of the method.

Background of the invention

Genetic modification is the process of deliberately creating changes in the genetic material of living cells with the purpose of modifying one or more genetically encoded biological properties of that cell, or of the organism of which the cell forms part or into which it can regenerate. These changes can take the form of deletion of parts of the genetic material, addition of exogenous genetic material, or changes in the existing nucleotide sequence of the genetic material. Methods for the genetic modification of eukaryotic organisms have been known for over 20 years, and have found widespread application in plant, human and animal cells and micro-organisms for improvements in the fields of agriculture, human health, food quality and environmental protection. The common methods of genetic modification consist of adding common methods of genetic modification consist of adding exogenous DNA fragments to the genome of a cell, which will then confer a new property to that cell or its organism over and above the properties encoded by already existing genes (including applications in which the expression of existing genes will thereby be suppressed). Although many such examples are effective in obtaining the desired properties, these methods are nevertheless not very precise, because there is no control over the genomic positions in which the exogenous DNA fragments are inserted (and hence over the ultimate levels of expression), and because the desired effect will have to manifest itself over the natural properties encoded by the original and well-balanced genome. On the contrary, methods of genetic modification that will result in the addition, deletion or conversion of nucleotides in predefined genomic loci will allow the precise modification of existing genes.

Oligonucleotide-directed Targeted Nucleotide Exchange (TNE, sometimes ODTNE) is a method, that is based on the delivery into the eukaryotic cell nucleus of synthetic oligonucleotides (molecules consisting of short stretches of nucleotide-like moieties that resemble DNA in their Watson-Crick basepairing properties, but may be chemically different from DNA) (Alexeev and Yoon, Nature Biotechnol. 16: 1343, 1998; Rice, Nature Biotechnol. 19: 321, 2001; Kmiec, J. Clin. Invest. 112: 632, 2003). By deliberately designing a mismatch nucleotide in the homology sequence of the oligonucleotide, the mismatch nucleotide may be incorporated in the genomic DNA sequence. This method allows the conversion of single or at most a few nucleotides in existing loci, but may be applied to create stop codons in existing genes, resulting in a disruption of their function, or to create codon changes, resulting in genes encoding proteins with altered amino acid composition (protein engineering).

Targeted nucleotide exchange (TNE) has been described in plant, animal and yeast cells. The first TNE reports utilized a so-called chimera that consisted of a self-complementary oligonucleotide that is designed to intercalate at the chromosomal target site. The chimera contains a mismatched nucleotide that forms the template for introducing the mutation at the chromosomal target. In order to select for TNE events, most studies attempt to introduce a single nucleotide change in an endogenous gene that leads to herbicide resistance. The first examples using chimeras came from human cells (see the review Rice et al. Nat. Biotech. 19: 321-326). The use of chimeras has also been successful in the plant species tobacco, rice, and maize (Beetham et al. 1999 Proc. Natl. Acad. Sci. USA 96: 8774-8778; Kochevenko et al. 2003 Plant Phys. 132: 174-184; Okuzaki et al. 2004 Plant Cell Rep. 22: 509-512). However, the activity of chimeras was found to be difficult to reproduce and so the TNE activity of single stranded (ss) oligonucleotides has been tested. These have been found to give more reproducible results in wheat, yeast and human cells (Liu et al. 2002 Nuc. Acids Res. 30: 2742-2750; review, Parekh-Olmedo et al. 2005 Gene Therapy 12: 639-646; Dong et al.2006 Plant Cell Rep. 25: 457-65).

Several groups have shown that TNE can also be detected using total cellular protein extracts. Such assays for TNE activity are called cell free assays (Cole-Strauss et al. 1999 Nucleic Acids Res. 27: 1323-1330; Gamper et al. 2000 Nucleic Acids Res. 28, 4332-4339; Kmiec et al. 2001 Plant J. 27: 267-274; Rice et al. 2001 40: 857-868). The assay is setup as follows. A plasmid containing two bacterial antibiotic resistance genes (kanamycin and carbenicillin) is mutated so that one of the antibiotic resistance genes (e.g. kanamycin) contains an in frame stop codon due to the alteration of a single nucleotide (e.g. TAT to TAG). This mutated plasmid is then incubated with total cellular protein and a single stranded oligonucleotide designed to correct the stop codon in the antibiotic resistance gene. The proteins necessary for TNE are present in the cellular extract and utilize the oligonucleotide to alter the stop codon in the antibiotic resistance gene, restoring the resistance phenotype. Plasmid DNA is then purified from the reaction mixture and transformed to *E. coli.* The bacteria are then plated out on media containing kanamycin and the number of bacterial colonies represents the number of TNE repair events. The electroporation efficiency is calculated by counting the number of colonies growing on media containing carbenicillin. The TNE efficiency can be quantified by calculating the ratio of repaired plasmids against the total number of transformed plasmids.

In such an experiment, the oligonucleotide effects a substitution, altering TAG to TAC. Furthermore, the cell free system can also be used to study the possibility of using oligonucleotides to produce single nucleotide insertions. Plasmids can be produced which have a single nucleotide deleted from the antibiotic resistance gene, generating a frame shift mutation. In the cell free assay, the deletion is repaired by addition of a nucleotide mediated by the oligonucleotide.

The greatest problem facing the application of TNE in cells of higher organisms such as plants is the low efficiency that has been reported so far. In maize Zhu et al. (2000 Nature Biotech. 18: 555-558) reported a conversion frequency of $1 \times 10^{-4}$. Subsequent studies in tobacco (Kochevenko et al. 2003 Plant Phys: 132: 174-184) and rice (Okuzaki et al. 2004 Plant Cell Rep. 22: 509-512) have reported frequencies of $1 \times 10^{-6}$ and $1 \times 10^{-4}$ respectively. These frequencies remain too low for the practical application of TNE.

Faithful replication of DNA is one of the key criteria that mediates maintenance of genome stability and ensures that the genetic information contained in the DNA is passed on free of mutation from one generation to the next. Many errors arise from damage in the parental DNA strand or are generated by agents that react with DNA bases (UV light, environmental toxins). Every organism must maintain a safeguard to prevent or correct these mutations. The mismatch repair system (MMR) is thought to recognize and correct mismatched or unpaired bases caused during DNA replication, in DNA damage surveillance and in prevention of recombination between non-identical sequences (Fedier and Fink, 2004 Int. J Oncol. 2004 ;24(4):1039-47), and contributes to the fidelity of DNA replication in living cells.

TNE has been described in a variety of patent applications of Kmiec, *inter alia* in WO0173002, WO03/027265, WO01/87914, WO99/58702, WO97/48714, WO02/10364. In WO 01/73002 it is contemplated that the low efficiency of gene alteration obtained using unmodified DNA oligonucleotides is largely believed to be the result of degradation of the donor oligonucleotides by nucleases present in the reaction mixture or the target cell. To remedy this problem, it is proposed to incorporate modified nucleotides that render the resulting oligonucleotides resistant against nucleases. Typical examples include nucleotides with phosphorothioate linkages, 2'-O-methyl-analogs or locked nucleic acids (LNAs). These modifications are preferably located at the ends of the oligonucleotide, leaving a central DNA domain surrounding the targeted base. Furthermore, the publication stipulates that specific chemical interactions are involved between the converting oligonucleotide and the proteins involved in the conversion. The effect of such chemical interactions to produce nuclease resistant termini using modifications other than LNA, phosphorothioate linkages or 2'-O-methyl analogue incorporation in the oligonucleotide is impossible to predict because the proteins involved in the alteration process and their chemical interaction with the oligonucleotide substituents are not yet known and, according to the inventors of WO0173002, cannot be predicted:

As the efficiency of the current methods of ODTNE is relatively low (as stated previously, between $10^{-6}$ and $10^{-4}$, despite reported high delivery rates of the oligonucleotide of 90%) there is a need in the art to come to methods for TNE that are more efficient. Accordingly, the present inventors have set out to improve on the existing TNE technology.

***Description of the invention***

The invention is as set-out in the claims. The present inventors have now found that by incorporating nucleotides into the donor oligonucleotide for TNE that are capable of binding more strongly to the acceptor DNA than the corresponding unmodified nucleotides like A, C, T, or G, the rate of TNE can be increased significantly. Without being bound by theory, the present inventors believe that by the incorporation of modified nucleotides into the donor oligonucleotide, the donor oligonucleotide binds more strongly to the acceptor DNA and, hence increases the ratio of TNE. The present inventors have found that oligonucleotides containing one or more LNAs at positions close to, but not (directly) adjacent to the mismatch, i.e. located at a distance of at least one nucleotide from the mismatch, improves the efficiency of TNE significantly.

To this end, the effect of using oligonucleotides incorporating one or more LNAs at various positions in the oligonucleotide on the frequency of TNE in the cell free system has been investigated. The TNE activity of such oligonucleotides was compared with the TNE activity of oligonucleotides made up of normal DNA. It was found that oligonucleotides containing one or more LNAs at positions removed at least one nucleotide from the mismatch increased the TNE efficiency for both substitutions and insertions in the cell free assay to a level hitherto unobserved. The oligonucleotides containing LNAs were up to 10 fold more efficient in the cell free assay compared to oligonucleotides made up of normal DNA. It was also found that the TNE efficiency could be improved with increasing the number of LNAs in the oligonucleotide whereby the LNAs are positioned at least 2 nucleotides apart, preferably at least 3, more preferably at least 4. Furthermore it was found that the improvement observed was locus independent, indicating that oligonucleotides of the invention with LNAs at particular positions compared to the mismatch are capable of providing enhanced frequencies of TNE in a species independent manner, such as in plant and animal cells.

The present invention is thus based on the inventive consideration that the desired targeted nucleotide exchange can be achieved by the use of partly (i.e. at most 75%, preferably at most 50%) LNA modified oligonucleotides. The location, type and amount of modification of the oligonucleotide can be varied within limits as will be disclosed herein below.

The present invention thus, in one aspect provides LNA modified oligonucleotides. The LNA modified, ss-oligonucleotides can be used to introduce specific genetic changes in plant and animal or human cells. The invention is applicable in the field of biomedical research, agriculture and to construct specifically mutated plants and animals, including humans. The invention is also applicable in the field of medicine and gene therapy.

The sequence of an oligonucleotide of the invention is homologous to the target strand except for the part that contains a mismatch base that introduces the base change in the target strand. The mismatched base is introduced into the target sequence. By manipulating the modification (compared to conventional A, C, T, or G) of the nucleotides, and more in particular, by manipulating the location and amount of LNA modification of the oligonucleotide that introduces the mismatch, the efficiency (or the degree of successful incorporation of the desired nucleotide at the desired position in the DNA duplex) can be improved.

Another aspect of the invention resides in a method for the targeted alteration of a parent DNA strand (first strand, second strand) by contacting the parent DNA duplex with an oligonucleotide that contains at least one mismatch nucleotide compared to the parent strand, wherein the donor oligonucleotide contains a section that is modified with LNA at particular positions to have a higher binding capacity than the parent (acceptor)strand in the presence of proteins that are capable of targeted nucleotide exchange.

Thus, the inventive gist of the invention lies in the improvement in the binding capacity, of the intercalating oligonucleotide (sometimes referred to as the donor) with LNA nucleotides relative to the unmodified intercalating oligonucleotide, whereby the LNA modification is located at one or more positions that are not adjacent to the mismatch.

### Detailed description of the invention

In one aspect the disclosure relates to an oligonucleotide for targeted alteration of a duplex DNA sequence, the duplex DNA sequence containing a first DNA sequence and a second DNA sequence which is the complement of the first DNA sequence, the oligonucleotide comprising a domain that is capable of hybridising to the first DNA sequence, which domain comprises at least one mismatch with respect to the first DNA sequence, and wherein the oligonucleotide comprises at least one section that contains at least one modified nucleotide having a higher binding affinity compared to naturally occurring A, C, T or G and wherein, preferably, the at least one modified nucleotide binds stronger to a nucleotide in an opposite position in the first DNA sequence as compared to a naturally occurring nucleotide complementary to the nucleotide in the opposite position in the first DNA sequence, wherein the at least one modified nucleotide is a LNA that is positioned at a distance of at.least one nucleotide from the at least one mismatch, and preferably wherein the oligonucleotide contains at most about 75% modified nucleotides.

In one aspect, the disclosure pertains to an LNA modified oligonucleotide for targeted alteration of a duplex DNA sequence. The duplex DNA sequence contains a first DNA sequence and a second DNA sequence. The second DNA sequence is the complement of the first DNA sequence and pairs to it to form a duplex. The oligonucleotide comprises a domain that comprises at least one mismatch with respect to the duplex DNA sequence to be altered. Preferably, the domain is the part of the oligonucleotide that is complementary to the first strand, including the at least one mismatch.

Preferably, the mismatch in the domain is with respect to the first DNA sequence. The oligonucleotide comprises a section that is modified with at least one LNA to have a higher binding affinity than the (corresponding part of the) second DNA sequence. Preferably the at least one modified nucleotide is a LNA that is positioned at a distance of at least one nucleotide from the at least one mismatch, more preferably the oligonucleotide contains at most about 75% LNA modified nucleotides.

The domain that contains the mismatch and the section containing the modified nucleotide(s) may be overlapping. Thus, in certain embodiments, the domain containing the mismatch is located at a different position on the oligonucleotide than the section of which the modification is considered. In certain embodiments, the domain incorporates the section. In certain embodiments the section can incorporate the domain. In certain embodiments the domain and the section are located at the same position on the oligonucleotide and have the same length i.e. they coincide in length and position. In certain embodiments, there can be more than one section within a domain.

For the present invention, this means that the part of the oligonucleotide that contains the mismatch which is to be incorporated in the DNA duplex can be located at a different or shifted position from the part of the oligonucleotide that is modified. In particular, in certain embodiments wherein the cell's repair system (or at least the proteins involved with this system, or at least proteins that are involved in TNE) determines which of the strands contain the mismatch and which strand is to be used as the template for the correction of the mismatch.

In certain embodiments, the oligonucleotide comprises a section that contains at least one, preferably at least 2, more preferably at least 3 LNA modified nucleotide(s). In certain embodiments, the section on the oligonucleotide can contain more than 4, 5, 6, 7, 8, 9, or 10 LNA modified nucleotides.

In certain embodiments, the at least one LNA is positioned at a distance of at most 10 nucleotides, preferably at most 8 nucleotides, more preferably at most 6 nucleotides, even more preferably at most 4, 3, or 2 nucleotides from the mismatch. In a more preferred embodiment the at least one LNA is positioned at a distance of 1 nucleotide from the mismatch, i.e. one nucleotide is positioned between the mismatch and the LNA. In certain embodiments relating to oligonucleotides containing more than one LNA, each LNA is located at a distance of at least one nucleotides from the mismatch. In a preferred embodiment, LNAs are not located adjacent to each other but are spaced apart by at least one nucleotide, preferably two or three nucleotides. In certain embodiments, in the case of two or more (even numbers of) LNA modifications of the oligonucleotide, the modifications are spaced at (about) an equal distance from the mismatch. In other words, preferably the LNA modifications are positioned symmetrically around the mismatch. For example, in a preferred embodiment, two LNAs are positioned symmetrically around the mismatch at a distance of 1 nucleotide from the mismatch (and 3 nucleotides from each other).

In certain embodiments, at most 50 % of the modified nucleotides of the oligonucleotide are LNA derivatives, i.e. the conventional A, C, or G is replaced by its LNA counterpart, preferably at most 40%, more preferably at most 30%, even more preferably at most 20%, and most preferably at most 10%.

In certain embodiments, more than one mismatch can be introduced, either simultaneously or successively. The oligonucleotide can accommodate more than one mismatch on either adjacent or removed locations on the oligonucleotide. To this end, the oligonucleotide can be adapted to accommodate a second set of LNAs that, follow the principles outlined herein, provided they do not interfere with each other's improved binding capacity due to the particular conformation of the LNAs in the oligonucleotide, i.e. preferably spaced around the mismatch at a distance of 1 nucleotide from the mismatch. In certain embodiments the oligonucleotide can comprise two, three, four or more mismatch nucleotides which may be adjacent or remote (i.e. non-adjacent). The oligonucleotide can comprise further domains and sections to accommodate this, and in particular can comprise several sections. In certain embodiments, the oligonucleotide may incorporate a potential insert that is to be inserted in the acceptor strand. Such an insert may vary in length from more than five up to 100 nucleotides. In a similar way in certain embodiments, deletions can be introduced of similar length variations (from 1 to 100 nucleotides).

In a further aspect of the disclosure, the design of the oligonucleotide can be achieved by:

- determining the sequence of the acceptor strand, or.at least of a section of the sequence around the nucleotide to be exchanged. This can typically be in the order of at least 10, preferably 15, 20, 25 or 30 nucleotides adjacent to the mismatch, preferably on each side of the mismatch, (for example GGGGGGXGGGGGG, wherein X is the mismatch);

- designing a donor oligonucleotide that is complementary to one or both the sections adjacent to the mismatch and contains the desired nucleotide to be exchanged (for example CCCCCCYCCCCCC);

- providing (e.g. by synthesis) the donor oligonucleotide with LNA modifications at desired positions. Modifications may vary widely, depending on the circumstances. Examples are $CCC^mCC^mCYCC^mCCC^mC$, $CCC^mCCCYCCC^m$-$CCC$, $CCCCCCYCCC^mC^mC^mC^m$, $C^mC^mC^mC^mC^mCYCCCCCC$, $CCCCC^mCYCCC^mCCCCC$, and so on, wherein $C^m$ stands for a LNA modified nucleotide residue. For a different acceptor sequence, e.g. ATGCGTACXGTCCATGAT, corresponding donor oligonucleotides can be designed, e.g. TACGCALGYCLGGTACTA (L= LNA) with modification as variable as outlined hereinbefore.

- subjecting the DNA to be modified with the donor oligonucleotide in the presence of proteins that are capable of targeted nucleotide exchange, for instance, and in particular, proteins that are functional in the mismatch repair mechanism of the cell.

Without being bound by theory, improved binding affinity is thought to increase the likelihood that an oligonucleotide finds and remains bound to its target, thus improving the TNE efficiency. Many different chemical modifications of the sugar backbone or the base confer improved binding affinity. The present inventors however, chose to focus on LNA modified oligonucleotides and found that their activity in TNE was dependent on the position in the oligonucleotide.

Locked Nucleic Acid (LNA) is a DNA analogue with very interesting properties for use in antisense gene therapy. LNAs are bicyclic and tricyclic nucleoside and nucleotide analogues and the oligonucleotides that contain such analogues. The basic structural and functional characteristics of LNAs and related analogues are disclosed in various publications and patents, including WO 99/14226, WO 00/56748, WO00/66604, WO 98/39352, United States Patent No.6, 043, 060, and United States Patent No. 6,268,490.

Specifically, it combines the ability to discriminate between correct and incorrect targets (high specificity) with very high bio-stability (low turnover) and unprecedented affinity (very high binding strength to target). In fact, the affinity increase recorded with LNA leaves the affinities of all previously reported analogues in the low-to-modest range.

LNA is an RNA analogue, in which the ribose is structurally constrained by a methylene bridge between the 2'-oxygen and the 4'-carbon atoms. This bridge restricts the flexibility of the ribofuranose ring and locks the structure into a rigid bicyclic formation. This so-called N-type (or 3'-endo) conformation results in an increase in the $T_m$ of LNA containing duplexes, and consequently higher binding affinities and higher specificities. NMR spectral studies have actually demonstrated the locked N-type conformation of the LNA sugar, but also revealed that LNA monomers are able to twist their unmodified neighbour nucleotides towards an N-type conformation. Importantly, the favourable characteristics of LNA do not come at the expense of other important properties as is often observed with nucleic acid analogues.

LNA can be mixed freely with all other chemistries that make up the DNA analogue universe. LNA bases can be incorporated into oligonucleotides as short all-LNA sequences or as longer LNA/DNA chimeras. LNAs can be placed in internal, 3' or 5'-positions. However, due to their rigid bicyclic conformations, LNA residues sometimes disturb the helical twist of nucleic acid strands. It is hence generally less preferred to design an oligonucleotide with two or more adjacent LNA residues. Preferably, the LNA residues are separated by at least one (modified) nucleotide that does not disturb the helical twist, such as a conventional nucleotide (A, C, T, or G).

The originally developed and preferred LNA monomer (the ß-D-oxy-LNA monomer) has been modified into new LNA monomers. The novel α-L-oxy-LNA shows superior stability against 3' exonuclease activity, and is also more powerful and more versatile than ß-D-oxy-LNA in designing potent antisense oligonucleotides. Also xylo-LNAs and L-ribo LNAs can be used, as disclosed in WO9914226, WO00/56748, WO00/66604. In the present invention, any LNA of the above types is effective in achieving the goals of the invention, i.e. improved efficiency of TNE, with a preference for β-D-LNA analogues.

In the art on TNE, LNA modification has been listed amongst a list of possible oligonucleotide modifications as alternatives for the chimeric molecules used in TNE. However, there is no indication in the art thus far that suggests that LNA modified single-stranded DNA oligonucleotides enhances TNE efficiency significantly to the extent that has presently been found when the LNA is positioned at least one nucleotide away from the mismatch and/or the oligonucleotide does not contain more than about 75 % (rounded to the nearest whole number of nucleotides) LNAs.

The delivery of the oligonucleotide can be achieved via electroporation or other conventional techniques that are capable of delivering either to the nucleus or the cytoplasm. In vitro testing of the method of the present invention can be achieved using the Cell Free system as is described i.a. in WO01/87914, WO03/027265, WO99/58702, WO01/92512.

As used herein, the capability of the donor oligonucleotide to influence the TNE depends on the type, location and number or relative amount of modified nucleotides that are incorporated in the donor oligonucleotide. This capability can be quantified for instance by normalising the binding affinity (or the binding energy (Gibbs Free Energy)) between conventional nucleotides at 1, i.e. for both AT and GC bindings, the binding affinity is normalised at 1. For the oligonucleotides used in the present invention the Relative Binding Affinity (RBA) of each modified nucleotide is > 1. This is exemplified in a formula below:

$$RBA = \sum_{n}^{1} RBA(modified) - \sum_{m}^{1} RBA(unmodified) > 0$$

Wherein RBA is the total relative binding affinity, RBA(modified) is the sum of the relative binding affinity of the modified.oligonucleotide with a length of n nucleotides and RBA(unmodified) is the sum of the relative binding affinity of the unmodified oligonucleotide with a length of m nucleotides. For example, an 100 bp oligonucleotide contains 10 modifications, each with a relative binding affinity of 1.1. The total RBA then equals: RBA = [(10*1.1) + (90*1.0)] - (100*1.0) = 1.

Note that the definition of RBA is in principle independent of the length of the nucleotide strand that is compared. However, when RBAs of different strands are compared it is preferred that the strands have about the same length or that sections of comparable length are taken. Note that RBA does not take into account that modifications can be grouped together on a strand. A higher degree of modification of a certain strand A compared to a strand B thus means that RBA(A) > RBA(B). For upstream and downstream sections, corresponding (local) RBA values may be defined and used. To accommodate the effect of the position of the modified nucleotide a weighing factor can be introduced into the RBA value. For instance, the effect of a modified nucleotide on the donor oligonucleotide adjacent to the mismatch can be larger than that of a modified nucleotide that is located at a distance five nucleotides removed from the mismatch. In the context of the present invention, RBA (Donor)> RBA (Acceptor).

In certain embodiments, the RBA value of the Donor may be at least 0.1 larger than the RBA of the Acceptor. In certain embodiments, the RBA value of the Donor may be at least 0.2, 0.3, 0.4, 0.5, 0.6, 0.7, 0.8, 0.9, 1.0, 1.5, 2.0, 2.5 larger than the RBA of the Acceptor. RBA values can be derived from conventional analysis of the modified binding affinity of the nucleotide, such as by molecular modelling, thermodynami.c measurements etc. Alternatively they can be determined by measurement of Tm differences between modified and unmodified strands. Alternatively, the RBA can be expressed as the difference in Tm between the unmodified and the modified strand, either by measurement or by calculation using conventional formulates for calculating the Tm of a set of nucleotides, or by a combination of calculation and measurements.

The donor oligonucleotides according to the disclosure may contain further modifications to improve the hybridisation characteristics such that the donor exhibits increased affinity for the target DNA strand so that intercalation of the donor is easier. The donor oligonucleotide can also be further modified to become more resistant against nucleases, to stabilise the triplex or quadruplex structure. Modification of the LNA modified donor oligonucleotides used in the invention can comprise phosphorothioate modification, 2-OMe substitutions, the use of further LNAs at the 3 and/or 5' termini of the oligonucleotide, PNAs (Peptide nucleic acids), ribonucleotide and other bases that modifies, preferably enhances, the stability of the hybrid between the oligonucleotide and the acceptor strand.

Particularly useful among such modifications are PNAs, which are oligonucleotide analogues where the deoxyribose backbone of the oligonucleotide is replaced by a peptide backbone. One such peptide backbone is constructed of repeating units of N- (2-aminoethyl) glycine linked through amide bonds. Each subunit of the peptide backbone is attached to a nucleobase (also designated "base"), which may be a naturally occurring, non-naturally occurring or modified base. PNA oligomers bind sequence specifically to complementary DNA or RNA with higher affinity than either DNA or RNA. Accordingly, the resulting PNA/DNA or PNA/RNA duplexes have higher melting temperatures (Tm). In addition, the Tm of the PNA/DNA or PNA/RNA duplexes is much less sensitive to salt concentration than DNA/DNA or DNA/RNA duplexes. The polyamide backbone of PNAs is also more resistant to enzymatic degradation. The synthesis of PNAs is described, for example, in WO 92/20702 and WO 92/20703. Other PNAs are illustrated, for example, in WO93/12129 and United States Patent No. 5,539,082, issued July 23, 1996. In addition, many scientific publications describe the synthesis of PNAs as well as their properties and uses. See, for example, Patel, Nature, 1993, 365, 490 ; Nielsen et al., Science, 1991, 254, 1497; Egholm, J. Am. Chem. Soc., 1992, 114, 1895; Knudson et al., Nucleic Acids Research, 1996,24,494; Nielsen et al., J. Am. Chem. Soc., 1996, 118, 2287 ; Egholm et al., Science, 1991,254,1497; Egholm et al., J. Am. Chem. Soc., 1992,114,1895; and Egholm et al., J Am. Chem. Soc., 1992,114,9677.

Useful further modifications of the LNA oligonucleotides are also known as Super A and Super T, obtainable from Epoch Biosciences Germany. These modified nucleotides contain an additional substituent that sticks into the major groove of the DNA where it is believed to improve base stacking in the DNA duplex.

In further embodiments, advantageous results can be achieved when, in addition to the LNA modified oligonucleotides, further modifications are introduced into oligonucleotide that enhance affinity of the oligonucleotide for the acceptor strand even more. Thus it has been found that LNA modified oligonucleotide used in the invention which further comprise C5-propyne modified pyrimidine and/or C7 propynyl modified purines improves the efficiency of TNE significantly.

The donor oligonucleotides of the disclosure can also be made chimeric, i.e. contain sections of DNA, RNA, LNA, PNA or combinations thereof.

Thus, in certain embodiments, the oligonucleotide further contains other; optionally non-methylated, modified nucleotides.

In certain embodiments, the oligonucleotide is resistant against nucleases. This is advantageous to prevent the oligonucleotide from being degraded by nucleases and enlarges the chance that the donor oligonucleotide can find its target (acceptor molecule).

In certain embodiments, the nucleotide in the oligonucleotide at the position of the mismatch can be modified. Whether or not the mismatch can be modified will depend to a large extent on the exact mechanism of the targeted nucleotide exchange or of the cell's DNA repair mechanism using the difference in affinity between the donor and acceptor strands. The same holds for the exact location of the other modified positions in the neighbourhood or vicinity of the mismatch. However, based on the disclosure presented herein, such an oligonucleotide can be readily designed and tested, taking into account the test procedures for suitable oligonucleotides as described herein elsewhere. In certain embodiments, the nucleotide at the position of the mismatch is not modified. In certain embodiments, modification is at a position one nucleotide away from to the mismatch, preferably 2, 3, 4, 5, 6 or 7 nucleotides away from the mismatch. In certain embodiments, modification is located at a position downstream from the mismatch. In certain embodiments, modification is located at a position upstream from the mismatch. In certain embodiments, the modification is located from 10 bp to 10kB from the mismatch; preferably from 50 to 5000 bp, more preferably from 100 to 500 from the mismatch.

The oligonucleotides that are used as donors can vary in length but generally vary in length between 10 and 500 nucleotides, with a preference for 11 to 100 nucleotides, preferably from 15 to 90, more preferably from 20 to 70 most preferably from 30 to 60 nucleotides.

In one aspect, the disclosure pertains to a method for the targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein a section of the donor oligonucleotide is modified with LNAs to express a higher degree of affinity to the first DNA sequence compared to an unmodified nucleotide at that position in the oligonucleotide, in the presence of proteins that are capable of targeted nucleotide exchange, wherein the LNA is positioned at a distance of at least one nucleotide vis-à-vis the mismatch.

The invention is, in its broadest form, generically applicable to all sorts of organisms such as humans, animals, plants, fish, reptiles, insects, fungi, bacteria and so on. The invention is applicable for the modification of any type of DNA, such as DNA derived from genomic DNA, linear DNA, artificial chromosomes, nuclear chromosomal DNA, organelle chromosomal DNA, BACs, YACs. The invention can be performed in vivo as well as ex vivo.

The invention is, in its broadest form, applicable for many purposes for altering a cell, correcting a mutation by restoration to wild type, inducing a mutation, inactivating an enzyme by disruption of coding region, modifying bioactivity of an enzyme by altering coding region, modifying a protein by disrupting the coding region.

The disclosure also relates to the use of oligonucleotides essentially as described hereinbefore, for altering a cell, correcting a mutation by restoration to wild type, inducing a mutation, inactivating an enzyme by disruption of coding region, modifying bioactivity of an enzyme by altering coding region, modifying a protein by disrupting the coding region, mismatch repair, targeted alteration of (plant) genetic material, including gene mutation, targeted gene repair and gene knockout.

The disclosure further relates to kits, comprising one or more oligonucleotides as defined herein elsewhere, optionally in combination with proteins that are capable of inducing MRM, and in particular that are capable of TNE.

Also disclosed is modified genetic material obtained by the method of the present invention, to cells and organisms that comprise the modified genetic material, to plants or plant parts that are so obtained.

The invention relates in particular to the use of the TNE method using the LNA modified oligonucleotides as disclosed herein to provide for herbicide resistance in plants. Also disclosed are plants that have been provided with resistance against herbicides, in particular glyphosate and/or sulfonylurea herbicides such as chlorsulfuron.

Description of the Figures

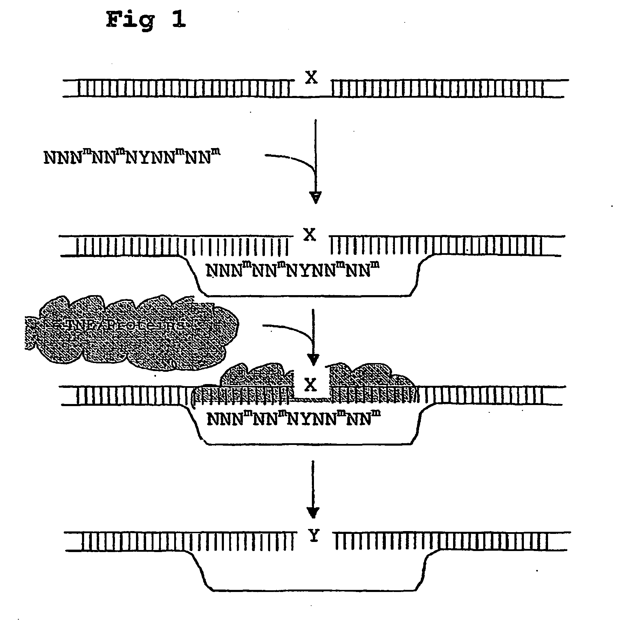

Figure 1: Schematic representation of targeted nucleotide exchange. An acceptor duplex DNA strand containing a nucleotide that is to be exchanged (X) is brought into contact with an LNA modified donor oligonucleotide (schematically given as NNN$^m$NNN$^m$YNN$^m$NN$^m$) containing the nucleotide to be inserted (Y). The triplex structure is subjected to or brought into contact with an environment that is capable of TNE or at least with proteins that are capable of performing TNE, such as are known as the cell-free enzyme mixture or a cell-free extract (see i.a. WO99/58702, WO01/73002).

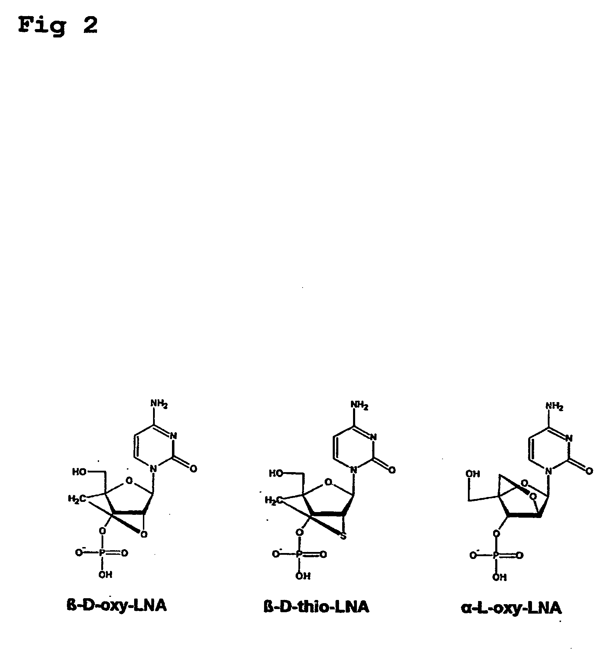

Figure 2: Chemical structures of various LNAs.

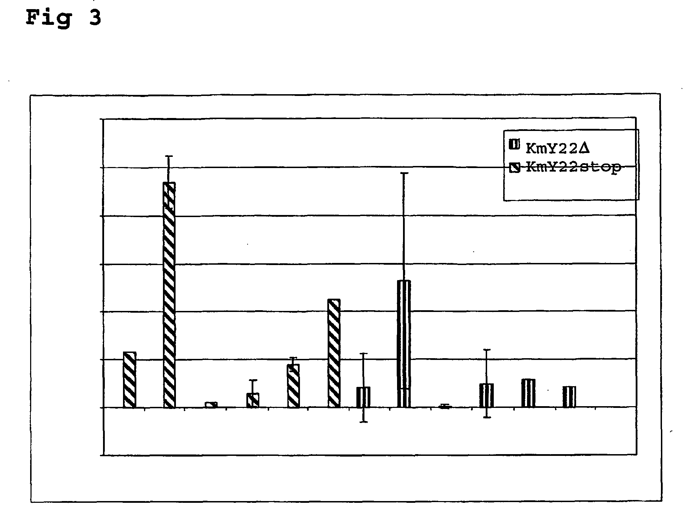

Figure 3: The TNE repair efficiency of oligonucleotides containing LNA modified nucleotides as measured using the cell free assay. Per experiment the repair efficiency using the normal DNA oligonucleotide was determined and set at a value of 1. The fold increase indicates the increase in repair seen using the β-D-LNA containing oligonucleotides compared to the repair efficiency obtained when using the normal DNA oligonucleotide.

Example

Materials and Methods

Oligonucleotides containing LNA nucleotides were purchased from Eurogentec. The sequences of the oligos used are shown below. The plasmid used in the experiments was a derivative of pCR2.1 (Invitrogen) that contains genes conferring both kanamycin and carbenicillin resistance. In frame stop codons and deletions were introduced into the kanamycin ORF and carbenicillin as previously described (Sawano et al. 2000 Nucleic Acids Res. 28: e78). Plasmid KmY22stop has a TAT to TAG mutation at codon Y22 in the kanamycin ORF. In plasmid KmY22. the third nucleotide of the Y22 codon (TA<u>T</u>) was deleted giving a frame shift.

Defective kanamycin genes and the oligonucleotides used in the cell free system:

| Km WT | GAG | AGG | CTA | TTC | GGC | TAT | GAC | TGG | GCA | CAA | CAG |
|---|---|---|---|---|---|---|---|---|---|---|---|
| | E | R | L | F | G | Y | D | W | | | |

(continued)

| KmY22stop | GAG | AGG | CTA | TTC | GGC | TAG | GAC | TGG | GCA | CAA | CAG |
|---|---|---|---|---|---|---|---|---|---|---|---|
| | E | R | L | F | G | * | | | | | |
| KmY22A | GAG | AGG | CTA | TTC | GGC | TA_ | GAC | TGG | GCA | CAA | CAG |
| | E | R | L | F | G | * | | | | | |

| Oligo | Sequence | LNA/DNA | SEQ ID |
|---|---|---|---|
| L1 | tgtgcccagtCgTagccgaatagc | 2/24 (8.3%) | 1 |
| L2 | tgtgcccagTcgtAgccgaatagc | 2/24 (8.3%) | 2 |
| L3 | TgTgCcCaGtCgTaGcCgAaTaGc | 12/24 (50%) | 3 |
| L4 | tGtGcCcAgTcGtAgCcGaAtAgC | 12/24 (50%) | 4 |
| L5 | tGtgcCcagTcgtAgccGaatAgc | 6/24 (25%) | 5 |
| L6 | tgtgCccagTcgtaGccgaAtagc | 4/24 (16.6%) | |

The relevant sequence of the kanamycin open reading frames and the amino acids encoded are shown. The single nucleotide mutations producing a stop codon (TAG, *) were introduced as previously described (Sawano et al. 2000 Nucleic Acids Res. 28: e78). The sequences of the LNA oligonucleotides used in the experiments are shown in uppercase. Oligonucleotides L1-L6 were used to convert the KmY22stop and KmY22Δ mutation to an alternative tyrosine encoding codon (TAC). The mismatch nucleotide in each oligonucleotide is underlined. The oligonucleotide binding regions are underlined on the kanamycin ORF. Oligonucleotides L1-L6 are complementary to the kanamycin coding sequence.

Cell free assays were performed as follows. Flower buds from *Arabidopsis thaliana* (ecotype Col-0) were collected and ground under nitrogen. 200μl protein isolation buffer (20mM HEPES pH7.5, 5mM KCl, 1.5mM MgCl$_2$, 10mM DTT, 10% (v/v) glycerol, 1% (w/v) PVP) was added. The plant debris was pelleted by centrifugation at 14k RPM for 30 mins and the supernatant was stored at -80°C. The protein concentration was measured using the NanoOrange Kit (Molecular Probes, Inc). A typical isolation resulted in a protein concentration of approximately 3-4μg/μl. The cell free reactions contained the following components. 1μg plasmid DNA (KmY22stop or KmY22Δ), 100ng of oligonucleotide, 30μg total plant protein, 4μl sheared salmon sperm DNA (3μg/μl), 2μl protease inhibitor mix (50x conc: Complete EDTA-free protease inhibitor cocktail tablets, Roche Diagnostics), 50μl 2x cell free reaction buffer (400mM Tris pH7.5, 200mM MgCl$_2$, 2mM DTT, 0.4mM spermidine, 50mM ATP, 2mM each CTP, GTP, UTP, 0.1mM each dNTPs and 10mM NAD) made up to a total volume of 100μl with water. The mixture was incubated at 37°C for 1 hr. The plasmid DNA was then isolated as follows. 100μl H$_2$O was added to each reaction to increase the volume followed by 200μl alkaline buffered phenol (pH8-10). This was vortexed briefly and then centrifuged and 13k rpm for 3 mins. The upper aqueous phase was transferred to a new tube and 200μl chloroform was then added. This was vortexed briefly, spun at 13k rpm for 3 mins and the aqueous phase transferred to a new tube. The DNA was precipitated by addition of 0.7 volume 2-propanol and the pellet resuspended in TE. To eliminate any co-purified oligonucleotide the DNA was passed over a Qiagen PCR purification column and the plasmid DNA eluted in a final volume of 30μl. 2μl of plasmid DNA was electroporated to 18μl of DH10B (Invitrogen) electrocompetent cells. After electroporation the cells were allowed to recover in SOC medium for 1hr at 37°C. After this period, kanamycin was added to a concentration of 100μg/ml and the cells were incubated for a further 3 hours. Solid media contained 100μg/ml kanamycin or carbenicillin. For the KmY22stop and KmY22. experiments, the number of TNE events were detected on kanamycin medium and the electroporation efficiency was calculated by counting the number of colonies obtained from a 10$^{-4}$ and 10$^{-5}$ dilution of the electroporation plated out on carbenicillin medium. The TNE efficiency was calculated by dividing the number of TNE events by the total number of transformed cells.

**Results**

The oligonucleotides were designed to produce a single nucleotide substitution (KmY22stop) or insertion (KmY22A) at the stop codon (TAG) introduced into the kanamycin ORF so that the codon again codes for the correct amino acid.

In each experiment unmodified DNA oligonucleotides and LNA modified oligonucleotides were run in parallel. In each experiment, the TNE efficiency obtained using the normal DNA oligonucleotide was arbitrarily set at 1 and the TNE efficiency of the LNA oligonucleotides was subsequently expressed as the fold increase over the normal DNA, oligonucleotide. We found that the increase in repair efficiency was dependent upon the position of the LNA's in the oligonucleotide. Oligonucleotide L1 contained LNA nucleotides flanking the mismatch nucleotide, and this showed no improvement over a DNA oligonucleotide. However, oligonucleotide L2, with the LNA nucleotides spaced one nucleotide further away from the mismatch, showed an average increase of 5 fold. Addition of extra LNA nucleotides (L3 & L4) decreases the repair efficiency below that of a normal DNA oligonucleotide to the level of background and these oligonucleotides are biologically inactive. This is confirmed by the data using L5 and L6. In L5, in addition to LNA's flanking the mismatch nucleotides as in L2, it also contains additional LNA nucleotides. In this case, the increase in repair obtained using L2 is not observed, presumably due to the inhibitory effects of the additional LNA nucleotides. L6 also shows improvement in repair when using KmY22stop. The same trend is observed when experiments are performed using KmY22Δ. Repair via single nucleotide insertions is known to be less efficient than repair via substitutions, and this is what is also observed here. L2 shows an increase in repair frequency, showing that also for insertions the position of the LNA nucleotides around the mismatch is an important feature to increase the repair frequency.

In the present disclosure, the experiments demonstrate that using an *in vitro* TNE assay, the cell free system, oligonucleotides containing LNA nucleotides show higher levels of TNE compared to the TNE efficiency obtained using normal DNA oligonucleotides. This enhancement can be as high as 5 fold. The effect is largely dependent upon the position of the LNA nucleotides relative to the mismatch nucleotide. In addition, the repair process is very sensitive to the number of LNA nucleotides on the oligonucleotide, when this reaches 50%, the oligonucleotide expresses a reduced biologically activity in the assay. Another form of LNA nucleotides, the 2'-amino-LNA analogs (Rosenbohm et al. (2003) Org Biomol Chem. 1, 655-663) can be further functionalized by addition of other chemical groups to the nucleotide. In addition to the increased binding affinity, such LNA nucleotides have additional chemical groups that can interact with DNA in many ways and further increase the binding affinity (Sorensen et al.. (2003) Chem Commun (Camb) 17, 2130-2131). Such 2'-amino-LNA may increase the further the repair above the 5 fold increase already shown.

**Claims**

1.  Method for targeted alteration of a duplex acceptor DNA sequence, comprising combining the duplex acceptor DNA sequence with a donor oligonucleotide, in the presence of proteins that are capable of targeted nucleotide exchange, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein the nucleotide at the mismatch is not modified, wherein the oligonucleotide contains more than one LNA, wherein each LNA is located at a distance of at least one nucleotide from the mismatch, and wherein the oligonucleotide comprises a section that contains two LNAs positioned symmetrically around the mismatch at a distance of one nucleotide from the mismatch, wherein the method is not for treatment of a human or animal body by surgery or therapy, and wherein the method is not for targeted alteration of DNA in humans.

2.  Method according to claim 1, wherein at most 50 % of the nucleotides of the oligonucleotide are LNA derivatives.

3.  Method according to claim 1 wherein in the oligonucleotide 2, and not more, nucleotides are LNA's.

4.  Method according to anyone of the previous claims, wherein the oligonucleotide has a length from 10 to 500 nucleotides.

5.  Method according to anyone of the previous claims, wherein the alteration is within a cell preferably selected from the group consisting of a plant cell, a fungal cell, a rodent cell, a primate cell, a human cell or a yeast cell.

6.  Method according to anyone of the claims 1 - 4, wherein the proteins are derived from a cell extract.

7.  Method according to claim 6, wherein the cell extract is selected from the group consisting of a plant cell extract, a fungal cell extract, a rodent cell extract, a primate cell extract, a human cell extract or a yeast cell extract.

8.  Method according to anyone of claims 1 - 4, wherein the alteration is a deletion, a substitution or an insertion of at least one nucleotide.

9.  Method according to anyone of the previous claims, wherein the target DNA is from fungi, bacteria, plants, mammals or humans.

10. Method according to anyone of the previous claims, wherein the duplex DNA is from genomic DNA, linear DNA, mammalian artificial chromosomes, bacterial artificial chromosomes, yeast artificial chromosomes, plant artificial chromosomes, nuclear chromosomal DNA, organelle chromosomal DNA, or episomal DNA.

11. Method according to anyone of the previous claims, for altering a cell, correcting a mutation by restoration to wild type, inducing a mutation, inactivating an enzyme by disruption of coding region, modifying bioactivity of an enzyme by altering coding region, or modifying a protein by disrupting the coding region.

12. Use of an oligonucleotide as defined in anyone of claims 1 - 4, for altering a cell, correcting a mutation by restoration to wild type, inducing a mutation, inactivating an enzyme by disruption of coding region, modifying bioactivity of an enzyme by altering coding region, modifying a protein by disrupting the coding region, mismatch repair, targeted alteration of (plant)genetic material, including gene mutation, targeted gene repair and gene knockout, wherein the use is not for treatment of a human or animal body by surgery or therapy, and wherein the use is not for targeted alteration of DNA in humans.

**Patentansprüche**

1. Verfahren zur gezielten Veränderung einer Duplex-Akzeptor-DNA-Sequenz, umfassend das Kombinieren der Duplex-Akzeptor-DNA-Sequenz mit einem Donor-Oligonucleotid, in Anwesenheit von Proteinen, die zum gezielten Nucleotidaustausch fähig sind, wobei die Duplex-Akzeptor-DNA-Sequenz eine erste DNA-Sequenz und eine zweite DNA-Sequenz, die das Komplement der ersten DNA-Sequenz ist, enthält und wobei das Donor-Oligonucleotid eine Domäne umfasst, die mindestens eine Fehlpaarung in Bezug auf die zu verändernde Duplex-Akzeptor-DNA-Sequenz umfasst, vorzugsweise in Bezug auf die erste DNA-Sequenz, und wobei das Nucleotid an der Fehlpaarung nicht modifiziert ist, wobei das Oligonucleotid mehr als eine LNA enthält, wobei sich jede LNA im Abstand von mindestens einem Nucleotid von der Fehlpaarung befindet, und wobei das Oligonucleotid einen Abschnitt umfasst, der zwei LNAs enthält, die im Abstand von einem Nucleotid von der Fehlpaarung symmetrisch um die Fehlpaarung herum angeordnet sind, wobei das Verfahren nicht zur Behandlung eines menschlichen oder tierischen Körpers durch Operation oder Therapie bestimmt ist und wobei das Verfahren nicht zur gezielten Veränderung von DNA bei Menschen bestimmt ist.

2. Verfahren gemäß Anspruch 1, wobei es sich bei höchstens 50% der Nucleotide des Oligonucleotids um LNA-Derivate handelt.

3. Verfahren gemäß Anspruch 1, wobei es sich bei 2, und nicht mehr, Nucleotiden in dem Oligonucleotid um LNAs handelt.

4. Verfahren gemäß einem der vorhergehenden Ansprüche, wobei das Oligonucleotid eine Länge von 10 bis 500 Nucleotiden aufweist.

5. Vefahren gemäß einem der vorhergehenden Ansprüche, wobei die Veränderung in einer Zelle stattfindet, vorzugsweise ausgewählt aus der Gruppe, bestehend aus einer Pflanzenzelle, einer Pilzzelle, einer Nagerzelle, einer Primatenzelle, einer menschlichen Zelle oder einer Hefezelle.

6. Vefahren gemäß einem der Ansprüche 1 - 4, wobei die Proteine aus einem Zellextrakt abgeleitet sind.

7. Verfahren gemäß Anspruch 6, wobei der Zellextrakt aus der Gruppe ausgewählt ist, bestehend aus einem Pflanzenzellextrakt, einem Pilzzellextrakt, einem Nagerzellextrakt, einem Primatenzellextrakt, einem menschlichen Zellextrakt oder einem Hefezellextrakt.

8. Verfahren gemäß einem der Ansprüche 1 - 4, wobei es sich bei der Veränderung um eine Deletion, eine Substitution oder eine Insertion von mindestens einem Nucleotid handelt.

9. Verfahren gemäß einem der vorhergehenden Ansprüche, wobei die Ziel-DNA von Pilzen, Bakterien, Pflanzen, Säugern oder Menschen stammt.

10. Verfahren gemäß einem der vorhergehenden Ansprüche, wobei die Duplex-DNA von genomischer DNA, linearer DNA, künstlichen Säugerchromosomen, künstlichen Bakterienchromosomen, künstlichen Hefechromosomen, künstlichen Pflanzenchromosomen, chromosomaler Kern-DNA, chromosomaler Organellen-DNA oder episomaler DNA stammt.

11. Verfahren gemäß einem der vorhergehenden Ansprüche zum Ändern einer Zelle, Korrigieren einer Mutation durch Wiederherstellung zum Wildtyp, Induzieren einer Mutation, Inaktivieren eines Enzyms durch Unterbrechung der kodierenden Region, Modifizieren der Bioaktivität eines Enzyms durch Verändern der kodierenden Region oder Modifizieren eines Proteins durch Unterbrechen der kodierenden Region.

12. Verwendung eines Oligonucleotids wie in einem der Ansprüche 1 - 4 definiert zum Ändern einer Zelle, Korrigieren einer Mutation durch Wiederherstellung zum Wildtyp, Induzieren einer Mutation, Inaktivieren eines Enzyms durch Unterbrechung der kodierenden Region, Modifizieren der Bioaktivität eines Enzyms durch Verändern der kodierenden Region oder Modifizieren eines Proteins durch Unterbrechen der kodierenden Region, zur Reparatur einer Fehlpaarung, zur gezielten Veränderung von genetischem (Pflanzen-)Material, einschließlich Genmutation, gezielter Genreparatur und Gen-Knockout, wobei die Verwendung nicht zur Behandlung eines menschlichen oder tierischen Körpers durch Operation oder Therapie bestimmt ist und wobei die Verwendung nicht zur gezielten Veränderung von DNA bei Menschen bestimmt ist.

**Revendications**

1. Procédé pour la modification ciblée d'une séquence d'ADN accepteur duplex, comprenant la combinaison de la séquence d'ADN accepteur duplex avec un oligonucléotide donneur, en présence de protéines qui sont capables d'effectuer un échange ciblé de nucléotides, dans lequel la séquence d'ADN accepteur duplex contient une première séquence d'ADN et une deuxième séquence d'ADN qui est le complément de la première séquence d'ADN et dans lequel l'oligonucléotide donneur comprend un domaine qui comprend au moins un mésappariement par rapport à la séquence d'ADN accepteur duplex à modifier, de préférence par rapport à la première séquence d'ADN, et dans lequel le nucléotide au niveau du mésappariement n'est pas modifié, dans lequel l'oligonucléotide contient plus d'un LNA, dans lequel chaque LNA est situé à une distance d'au moins un nucléotide du mésappariement, et dans lequel l'oligonucléotide comprend une section qui contient deux LNAs placés de façon symétrique autour du mésappariement à une distance d'un nucléotide du mésappariement, dans lequel le procédé n'est pas destiné au traitement d'un organisme humain ou animal par chirurgie ou thérapie, et dans lequel le procédé n'est pas destiné à une modification ciblée de l'ADN chez les humains.

2. Procédé selon la revendication 1, dans lequel au plus 50 % des nucléotides de l'oligonucléotide sont des dérivés de LNA.

3. Procédé selon la revendication 1, dans lequel dans l'oligonucléotide, 2 nucléotides, et pas plus, sont des LNAs.

4. Procédé selon l'une quelconque des revendications précédentes, dans lequel l'oligonucléotide a une longueur de 10 à 500 nucléotides.

5. Procédé selon l'une quelconque des revendications précédentes, dans lequel la modification est à l'intérieur d'une cellule choisie de préférence dans le groupe constitué par une cellule végétale, une cellule fongique, une cellule de rongeur, une cellule de primate, une cellule humaine ou une cellule de levure.

6. Procédé selon l'une quelconque des revendications 1 à 4, dans lequel les protéines sont dérivées d'un extrait cellulaire.

7. Procédé selon la revendication 6, dans lequel l'extrait cellulaire est choisi dans le groupe constitué par un extrait de cellules végétales, un extrait de cellules fongiques, un extrait de cellules de rongeur, un extrait de cellules de primate, un extrait de cellules humaines ou un extrait de cellules de levure.

8. Procédé selon l'une quelconque des revendications 1 à 4, dans lequel la modification est une délétion, une substitution ou une insertion d'au moins un nucléotide.

9. Procédé selon l'une quelconque des revendications précédentes, dans lequel l'ADN cible est issu de champignons, de bactéries, de plantes, de mammifères ou d'humains.

10. Procédé selon l'une quelconque des revendications précédentes, dans lequel l'ADN duplex provient d'un ADN génomique, d'un ADN linéaire, de chromosomes artificiels de mammifères, de chromosomes artificiels bactériens, de chromosomes artificiels de levure, de chromosomes artificiels de plantes, d'un ADN chromosomique nucléaire, d'un ADN chromosomique d'organite, ou d'un ADN épisomique.

11. Procédé selon l'une quelconque des revendications précédentes, destiné à modifier une cellule, corriger une mutation par la restauration du type sauvage, induire une mutation, inactiver une enzyme par la désorganisation de la région codante, modifier la bioactivité d'une enzyme par l'altération de la région codante, ou modifier une protéine par la désorganisation de la région codante.

12. Utilisation d'un oligonucléotide selon l'une quelconque des revendications 1 à 4, pour la modification d'une cellule, la correction d'une mutation par la restauration du type sauvage, l'induction d'une mutation, l'inactivation d'une enzyme par la désorganisation de la région codante, la modification de la bioactivité d'une enzyme par l'altération de la région codante, la modification d'une protéine par la désorganisation de la région codante, la réparation d'un mésappariement, la modification ciblée d'un matériel génétique (végétal), incluant une mutation de gène, une réparation de gène et une inactivation de gène ciblées, dans laquelle l'utilisation n'est pas destinée au traitement d'un organisme humain ou animal par chirurgie ou thérapie, et dans laquelle l'utilisation n'est pas destinée à la modification ciblée de l'ADN chez les humains.

## Fig 1

# Fig 2

β-D-oxy-LNA          ß-D-thio-LNA          α-L-oxy-LNA

# Fig 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0173002 A
- WO 03027265 A
- WO 0187914 A
- WO 9958702 A
- WO 9748714 A
- WO 0210364 A
- WO 9914226 A
- WO 0056748 A
- WO 0066604 A
- WO 9839352 A
- US 6043060 A
- US 6268490 B
- WO 0192512 A
- WO 9220702 A
- WO 9220703 A
- WO 9312129 A
- US 5539082 A
- NL 2006000244 W
- NL 2005000884 W

### Non-patent literature cited in the description

- **ALEXEEV ; YOON.** *Nature Biotechnol.,* 1998, vol. 16, 1343
- **RICE.** *Nature Biotechnol.,* 2001, vol. 19, 321
- **KMIEC.** *J. Clin. Invest.,* 2003, vol. 112, 632
- **RICE et al.** *Nat. Biotech.,* vol. 19, 321-326
- **BEETHAM et al.** *Proc. Natl. Acad. Sci. USA,* 1999, vol. 96, 8774-8778
- **KOCHEVENKO et al.** *Plant Phys.,* 2003, vol. 132, 174-184
- **OKUZAKI et al.** *Plant Cell Rep.,* 2004, vol. 22, 509-512
- **LIU et al.** *Nuc. Acids Res.,* 2002, vol. 30, 2742-2750
- **PAREKH-OLMEDO et al.** *Gene Therapy,* 2005, vol. 12, 639-646
- **DONG et al.** *Plant Cell Rep.,* 2006, vol. 25, 457-65
- **COLE-STRAUSS et al.** *Nucleic Acids Res.,* 1999, vol. 27, 1323-1330
- **GAMPER et al.** *Nucleic Acids Res.,* 2000, vol. 28, 4332-4339
- **KMIEC et al.** *Plant J.,* 2001, vol. 27, 267-274
- **RICE et al.** *PLANT J.,* 2001, vol. 40, 857-868
- **ZHU et al.** *Nature Biotech.,* 2000, vol. 18, 555-558
- **KOCHEVENKO et al.** *Plant Phys,* 2003, vol. 132, 174-184
- **FEDIER ; FINK.** *Int. J Oncol.,* 2004, vol. 24 (4), 1039-47
- **PATEL.** *Nature,* 1993, vol. 365, 490
- **NIELSEN et al.** *Science,* 1991, vol. 254, 1497
- **EGHOLM.** *J. Am. Chem. Soc.,* 1992, vol. 114, 1895
- **KNUDSON et al.** *Nucleic Acids Research,* 1996, vol. 24, 494
- **NIELSEN et al.** *J. Am. Chem. Soc.,* 1996, vol. 118, 2287
- **EGHOLM et al.** *Science,* 1991, vol. 254, 1497
- **EGHOLM et al.** *J. Am. Chem. Soc.,* 1992, vol. 114, 1895
- **EGHOLM et al.** *J Am. Chem. Soc.,* 1992, vol. 114, 9677
- **SAWANO et al.** *Nucleic Acids Res.,* 2000, vol. 28, 78
- **SAWANO et al.** *Nucleic Acids Res.,* 2000, vol. 28, e78
- **ROSENBOHM et al.** *Org Biomol Chem.,* 2003, vol. 1, 655-663
- **SORENSEN et al.** *Chem Commun (Camb),* 2003, vol. 17, 2130-2131

SEQUENCE LISTING

<110> Keygene NV

<120> Improved targeted nucleotide exchange with LNA modified oligonucleotides

<130> P27711PC03

<150> PCT/NL2006/000244
<151> 2006-05-09

<150> PCT/NL2005/000884
<151> 2005-12-22

<160> 6

<170> PatentIn version 3.3

<210> 1
<211> 24
<212> DNA
<213> artificial

<220>
<223> LNA modified TNE oligonucleotide

<220>
<221> modified_base
<222> (11)..(11)
<223> LNA-Cytosine

<220>

<221> modified_base
<222> (13)..(13)
<223> LNA-Tyrosine

<400> 1
tgtgcccagt cgtagccgaa tagc    24

<210> 2
<211> 24
<212> DNA
<213> artificial

<220>
<223> LNA modified TNE oligonucleotide

<220>
<221> modified_base
<222> (10)..(10)
<223> LNA- Tyrosine

<220>
<221> modified_base

<222> (14)..(14)
<223> LNA- adenine

<400> 2
tgtgcccagt cgtagccgaa tagc    24

<210> 3
<211> 24
<212> DNA
<213> artificial

<220>
<223> LNA modified TNE Oligonucleotide

<220>
<221> modified base
<222> (1)..(1)
<223> LNA-Tyrosine

<220>
<221> modified_base
<222> (3)..(3)
<223> LNA-Tyrosine

<220>
<221> modified_base
<222> (5)..(5)
<223> LNA-Cytosine

<220>
<221> modified base
<222> (7)..(7)
<223> LNA-Cytosine

<220>

<221> modified_base
<222> (9)..(9)
<223> LNA-Guanine

<220>
<221> modified_base
<222> (11)..(11)
<223> LNA-Cytosine

<220>
<221> modified_base
<222> (13)..(13)
<223> LNA-Tyrosine

<220>
<221> modified_base
<222> (15)..(15)
<223> LNA-Guanine

<220>
<221> modified_base
<222> (17)..(17)
<223> LNA-Cytosine

<220>
<221> modified_base
<222> (19)..(19) <223> LNA-Adenine

<220>
<221> modified_base
<222> (21)..(21)
<223> LNA-Tyrosine

<220>
<221> modified_base
<222> (23)..(23)
<223> LNA-Guanine

<400> 3
tgtgcccagt cgtagccgaa tagc      24

<210> 4
<211> 24
<212> DNA
<213> artificial

<220>
<223> LNA modified TNE oligonucleotide

<220>
<221> modified_base
<222> (2)..(2)
<223> LNA-Guanine

<220>
<221> modified_base
<222> (4)..(4)
<223> LNA-Guanine

<220>
<221> modified_base
<222> (6)..(6)
<223> LNA-Cytosine

<220>
<221> modified_base
<222> (8)..(8)
<223> LNA-Adenine

<220>
<221> modified_base
<222> (10)..(10)
<223> LNA-Tyrosine

<220>
<221> modified_base
<222> (12)..(12)
<223> LNA-Guanine

<220>
<221> modified_base
<222> (14)..(14)
<223> LNA-Adenine

<220>
<221> modified_base
<222> (16)..(16)
<223> LNA-Cytosine

<220>
<221> modified_base
<222> (18)..(18)
<223> LNA-Guanine

<220>
<221> modified_base
<222> (20)..(20)
<223> LNA-Adenine

<220>
<221> modified_base
<222> (22)..(22)
<223> LNA-Adenine

<220>

<221> modified_base
<222> (24)..(24)
<223> LNA-Cytosine

<400> 4
tgtgcccagt cgtagccgaa tagc        24

<210> 5
<211> 24
<212> DNA
<213> artificial

<220>
<223> LNA modified TNE oligonucleotide

<220>
<221> modified_base
<222> (2)..(2)
<223> LNA-Guanine

<220>
<221> modified_base
<222> (6)..(6)
<223> LNA-Cytosine

<220>
<221> modified_base

<222> (10)..(10)
<223> LNA-Tyrosine

<220>
<221> modified_base

<222> (14)..(14)
<223> LNA-Adenine

<220>
<221> modified_base
<222> (18)..(18)
<223> LNA-Guanine

<220>
<221> modified_base
<222> (22) .. (22)
<223> LNA-Adenine

<400> 5
tgtgcccagt cgtagccgaa tagc        24

<210> 6
<211> 24
<212> DNA
<213> artificial

<220>
<223> LNA modified TNE oligonucleotide

<220>
<221> modified_base
<222> (5)..(5)
<223> LNA-Cytosine

<220>
<221> modified_base
<222> (10)..(10)
<223> LNA-Tyrosine

<220>
<221> modified_base

<220>
<221> modified_base
<222> (15)..(15)
<223> LNA-Guanine

<220>
<221> modified_base
<222> (20)..(20)
<223> LNA-Adenine

<400> 6
tgtgcccagt cgtagccgaa tagc    24